(19) Europäisches Patentamt
European Patent Office
Office européen des brevets

(11) Publication number: **0 044 646 B1**

# (12) EUROPEAN PATENT SPECIFICATION

(45) Date of publication of the patent specification: **18.04.84**

(51) Int. Cl.³: **C 07 C 121/75**, C 09 K 3/34 // C07C63/04

(21) Application number: **81303036.8**

(22) Date of filing: **03.07.81**

(54) **4-(Trans-4'-alkylcyclohexyl)benzoic acid 4'''-cyano-4''-biphenylyl esters and liquid crystal compositions containing them.**

(30) Priority: **15.07.80 JP 96475/80**

(43) Date of publication of application: **27.01.82 Bulletin 82/4**

(45) Publication of the grant of the patent: **18.04.84 Bulletin 84/16**

(84) Designated Contracting States: **CH DE GB LI**

(56) References cited:
**EP - A - 0 003 215**
**EP - A - 0 012 820**

(73) Proprietor: **Chisso Corporation, 6-32, Nakanoshima 3-chome Kita-ku, Osaka-shi Osaka-fu (JP)**

(72) Inventor: **Fukui, Masahiro, 2126-5, Kamariyacho Kanazawaku, Yokohamashi Kanagawaken (JP)**
Inventor: **Inoue, Hiromichi, 10-2, Otsutomocho Kanazawaku, Yokohamashi Kanagawaken (JP)**
Inventor: **Goto, Yasuyuki, 10-2, Otsutomocho Kanazawaku, Yokohamashi Kanagawaken (JP)**

(74) Representative: **Ruffles, Graham Keith et al, MARKS & CLERK 57-60 Lincoln's Inn Fields, London WC2A 3LS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



ACTORUM AG

# 4-(Trans-4′-alkylcyclohexyl)benzoic Acid 4‴-Cyano-4″-biphenylyl Esters and Liquid Crystal Compositions containing them

This invention relates to novel organic compounds. More particularly it relates to novel liquid crystal compounds useful as a component of liquid crystal materials.

Liquid crystal substances have been widely used for liquid crystal display elements of the so-called twisted nematic mode utilizing nematic liquid crystals of a twisted liquid crystal arrangement. They have also been used for colour display elements employing a liquid crystal or a mixture of liquid crystals containing a suitable dyestuff and utilizing a guest-host effect, further DSM display elements utilizing a dynamic scattering effect, display elements utilizing a cholesteric phase-nematic phase transition, DAP type display elements utilizing an electrically controlled birefringence effect, etc.

As for liquid crystal materials used for these display elements, no single compound has been found whose various characteristic properties such as liquid crystal temperature range, actuation voltage, response performance, etc., are adequate for lasting practical use; hence it is the present state of the art that several kinds of liquid crystal compounds are mixed together to obtain compositions which endure practical use.

The object of the present invention is to provide compounds useful as components in liquid crystal compositions having practical superior characteristic properties and also a good stability.

The present invention provides—

4-(Trans-4′-alkylcyclohexyl)benzoic acid 4‴-cyano-4″-biphenylyl esters expressed by the general formula—

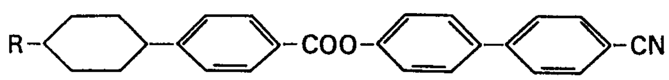

(I)

(wherein R represents an alkyl group having 1 to 10 carbon atoms, preferably 2 to 7, for example 5 or 7 carbon atoms) and liquid crystal compositions containing such esters.

The compounds of the present invention have a positive dielectric anisotropy, a broad liquid crystal temperature range, a very high transparent point, and a superior stability, but due to their high melting points, they cannot be singly used. However, they have a good compatibility with other liquid crystal compounds of various groups such as liquid crystals of the Schiff's base group, azoxy group, benzoic acid phenyl ester group, cyclohexanecarboxylic acid cyclohexyl ester group, biphenyl group, phenylcyclohexane group, phenylpyrimidine group, phenylmetadioxane group, etc., especially 4-alkyl- or 4-alkoxy-4′-cyanobiphenyls. When they are mixed with these other liquid crystals, they are useful as a high-temperature liquid crystal component having a function of raising the transparent points of liquid crystal compositions to broaden their liquid crystal temperature ranges. Such an effectiveness of the compounds of the present invention is greater than observed with 4-cyano-4″-pentyl-p-terphenyl, 4-cyano-4′-(trans-4″-pentylcyclohexyl)biphenyl, etc., which have so far been well known as compounds having a similar function.

The compounds of the present invention can be prepared according to the following steps—

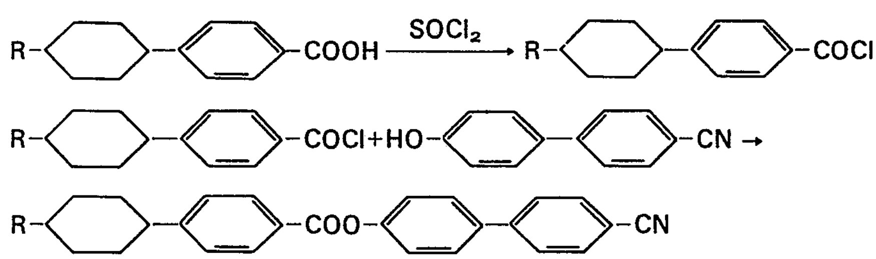


(I)

Namely, a 4-(trans-4′-alkylcyclohexyl)benzoic acid is reacted with thionyl chloride to obtain its acid chloride, which is then reacted with 4-hydroxy-4′-cyanobiphenyl in pyridine to obtain a desired 4-(trans-4′-alkylcyclohexyl)benzoic acid 4‴-cyano-4″-biphenylyl ester. Other esterification reactions can be used if desired in place of the acid chloride-alcohol reaction.

4-(Trans-4′-alkylcyclohexyl)benzoic acids as raw material can be prepared for example through the following reaction course—

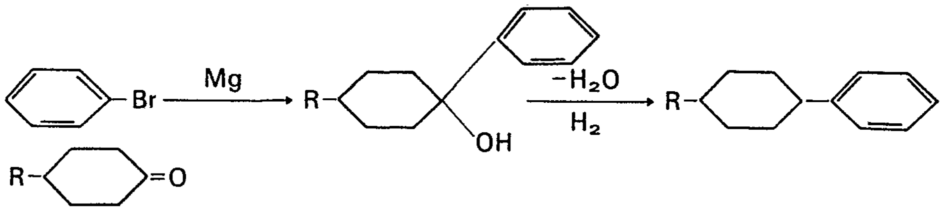

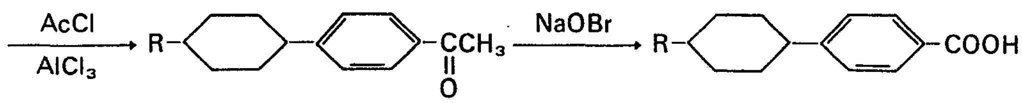


Monobromobenzene and a 4-alkylcyclohexanone are subjected to a Grignard reaction to obtain a 4-alkyl-1-phenylcyclohexanol, which is then subjected to hydrogenation in ethanol in the presence of a Raney nickel catalyst to obtain a mixture of cis- and trans-forms of the 4-alkyl-1-phenylcyclohexane. This mixture is distilled under reduced pressure and then subjected to a Friedel-Crafts reaction together with acetyl chloride in the presence of aluminum chloride employing methylene chloride as solvent to obtain a 4-(4'-alkylcyclohexyl)acetophenone. Aluminum chloride is removed from this product in a conventional way and the resulting material is distilled under reduced pressure to obtain a 4-(trans-4'-alkylcyclohexyl)-acetophenone separated from its cis-form, which is then subjected to an oxidation reaction with sodium hypobromite in a mixed solvent of water/dioxane at room temperature for several hours, followed by acidifying the reaction system to deposit crystals, which are then recrystallized from acetic acid to obtain the 4-(trans-4'-alkylcyclohexyl)benzoic acid.

The preparation of the compounds of the present invention and instances of their use will be described below in detail by way of the following non-limiting examples.

*Example 1:*

*Preparation of 4-(trans-4'-pentylcyclohexyl)benzoic acid 4'''-cyano-4''-biphenylyl ester*

4-(Trans-4'-pentylcyclohexyl)benzoic acid (7 g) and thionyl chloride (4.6 g) were fed into a 100 ml capacity eggplant-shaped flask and dissolved together on heating and further subjected to reflux for 30 min, followed by distilling off excess thionyl chloride and dissolving the resulting residue in 20 ml of toluene. On the other hand, 4-hydroxy-4'-cyanobiphenyl (5 g) and dry pyridine (10 g) were fed into a 200 ml capacity three-neck flask and dissolved together with stirring, followed by adding 50 ml of dry toluene and stirring to obtain a uniform solution. To this solution was gradually added dropwise through a dropping funnel a toluene solution of the acid chloride of 4-(trans-4'-pentylcyclohexyl)benzoic acid obtained above. After the addition, the contents were warmed to 60°C on a water bath, kept for 1½ h, and cooled. Toluene (200 ml) and water (20 ml) were added, and the mixture transferred into a separating funnel, followed by washing with an acid and then an alkali, further washing with water and making the liquid neutral. The resulting toluene layer was separated and active carbon (2 g) added, followed by warming up to 70°C, keeping for about 15 min, cooling, and removing the active carbon by suction-filtering. Water was azeotropically removed from the toluene solution by distilling off a part of toluene under the atmospheric pressure, followed by passing the resulting liquid through an activated alumina layer, completely distilling off toluene and recrystallizing the remaining solid from benzene (5 ml) to obtain colourless, acicular crystals as the desired product (4.8). The crystals had a melting point (C-N point) of 128°C and a transparent point of 280°C or higher (the probable value through extrapolation by means of mix-melting with a liquid crystal of cyano-biphenyl group corresponds to 460°C). Further the elemental analysis values of this product accorded well with the theoretical values as follows—

| | Analytical Value (%) | Theoretical Value (%) (as $C_{31}H_{33}NO_2$) |
|---|---|---|
| C | 82.7 | 82.45 |
| H | 7.2 | 7.37 |
| O | 7.2 | 7.09 |

*Examples 2 to 5:*

Example 1 was repeated, except that four 4-(trans-4'-alkylcyclohexyl)benzoic acids each having a different alkyl group were used in place of the 4-(trans-4'-pentylcyclohexyl)benzoic acid in Example 1, thereby to obtain ester compounds of the present invention corresponding to the four other benzoic acids. Each of these esters had colourless, acicular crystals. Their C-N point and N-I point were as follows—

*Example 2:*

*4-(Trans-4'-propylcyclohexyl)benzoic acid 4'''-cyano-4''-biphenylyl ester*

C-N point: 165°C, N-I point: 280°C or higher.

*Example 3:*

*4-(Trans-4'-heptylcyclohexyl)benzoic acid 4'''-cyano-4''-biphenylyl ester*

m.p. (C-S point): 115°C, S-N point: 209°C, N-I point: 280°C or higher (the probable value through the extrapolation as in Example 1 was about 420°C).

*Example 4:*

*4-(Trans-4'-ethylcyclohexyl)benzoic acid 4'''-cyano-4''-biphenylyl ester*

C-N point: 164°C, N-I point: 280°C or higher.

*Example 5:*

*4-(Trans-4'-butylcyclohexyl)benzoic acid 4'''-cyano-4''-biphenylyl ester*

C-N point: 129°C, N-I point: 280°C or higher.

*Example 6:*

A liquid crystal composition having the following composition was prepared—

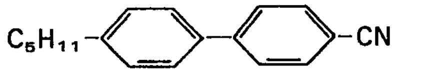
45% by weight

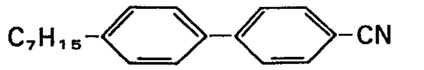
30% by weight

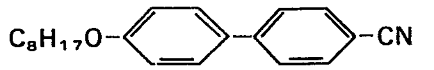
17% by weight

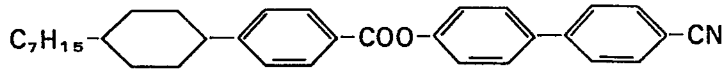
8% by weight

This liquid crystal composition had a nematic liquid crystal temperature range of −10 to +66°C and a viscosity of 48 cPo at 20°C. This composition was sealed between two glass base plates equipped with transparent electrodes each coated with $SiO_2$ film and subjected to a surface orientation treatment by rubbing to prepare a TN cell of 10 μm thick. The actuation voltages were measured, giving a threshold voltage of 1.6 V and a saturation voltage of 2.2 V.

For comparison, 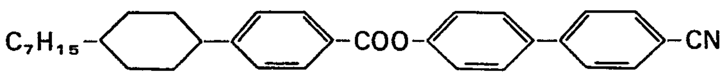

contained in this composition was replaced by 30% by weight 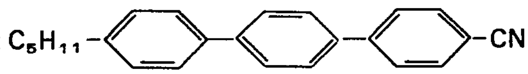

which has been often used up until now. The resulting composition had a nematic liquid crystal range as narrow as −10 to 58°C and a viscosity of 42 cPo at 20°C. Its threshold voltage and saturation voltage measured under the same conditions as above were 1.6 and 2.2 V, respectively.

*Example 7:*

A liquid crystal composition having the following composition was prepared—

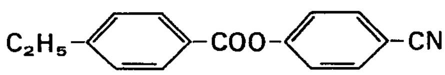
15% by weight

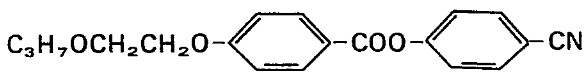
10% by weight

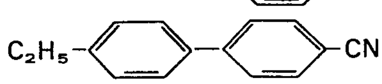
25% by weight

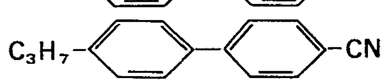
20% by weight

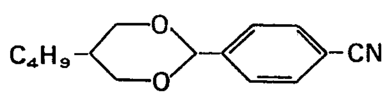
20% by weight

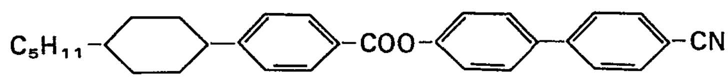
5% by weight

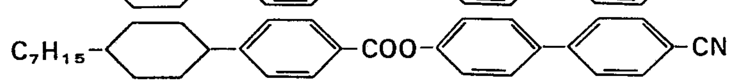
5% by weight

Its nematic liquid crystal range was measured as −10 to +57.5°C. Its threshold voltage and saturation voltage obtained by sealing the composition in a TN cell as in Example 6 were 1.00 and 1.45 V, respectively.

## Claims

1. A 4-(trans-4′-alkylcyclohexyl)benzoic acid 4‴-cyano-4″-biphenylyl ester expressed by the general formula—

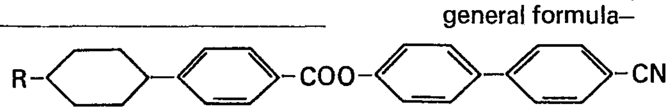 (I)

wherein R represents an alkyl group having 1 to 10 carbon atoms.

2. An ester according to Claim 1, wherein R is an alkyl group having 2 to 7 carbon atoms.

3. An ester according to Claim 1, wherein R is $C_5H_{11}$.

4. An ester according to Claim 1, wherein R is $C_7H_{15}$.

5. A liquid crystal composition based on a mixture of liquid crystal compounds, characterized in that the mixture contains at least one ester of the general formula (I), as defined in Claim 1.

6. A liquid crystal composition according to Claim 5, further comprising at least one 4-alkyl-4'-cyanobiphenyl.

7. A liquid crystal composition according to Claim 5, further comprising at least one 4-alkoxy-4'-cyanobiphenyl.

**Revendications**

1. Un 4'''-cyano-4''-biphénylylester d'acide 4-(trans-4'-alkycyclohexyl)benzoïque, représenté par la formule générale:

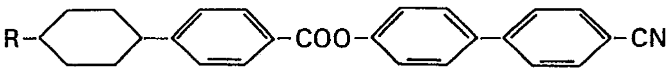 (I)

dans laquelle R représente un groupe alkyle ayant 1 à 10 atomes de carbone.

2. Un ester selon la revendication 1, dans lequel R est un groupe alkyle ayant 2 à 7 atomes de carbone.

3. Un ester selon la revendication 1, dans lequel R est $C_5H_{11}$.

4. Un ester selon la revendication 1, dans lequel R est $C_7H_{15}$.

5. Une matière cristalline liquide à base d'un mélange de composés à cristaux liquides, caractérisée en ce que le mélange contient au moins un ester de formule générale (I), comme défini dans la revendication 1.

6. Une matière cristalline liquide selon la revendication 5, comprenant, en outre, au moins un 4-alkyl-4'-cyanobiphényle.

7. Une matière cristalline liquide selon la revendication 5, comprenant, en outre, au moins un 4-alkoxy-4'-cyanobiphényle.

**Patentansprüche**

1. 4-(Trans-4'-alkylcyclohexyl)benzoesäure-4'''-cyano-4''-biphenylylester der allgemeinen Formel:

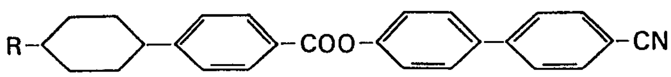 (I)

worin R eine Alkylgruppe mit 1 bis 10 Kohlenstoffatomen darstellt.

2. Ester nach Anspruch 1, wobei R eine Alkylgruppe mit 2 bis 7 Kohlenstoffatomen ist.

3. Ester nach Anspruch 1, wobei R $C_5H_{11}$ ist.

4. Ester nach Anspruch 1, wobei R $C_7H_{15}$ ist.

5. Flüssigkristallzusammensetzung auf der Basis eines Gemischs von Flüssigkristallkomponenten, dadurch gekennzeichnet, dass das Gemisch wenigstens einen Ester der allgemeinen Formel (I) nach Anspruch 1 enthält.

6. Flüssigkristallzusammensetzung nach Anspruch 5, die ausserdem wenigstens ein 4-Alkyl-4'-cyanobiphenyl umfasst.

7. Flüssigkristallzusammensetzung nach Anspruch 5, die ausserdem wenigstens ein 4-Alkoxy-4'-cyanobiphenyl umfasst.